# EUROPEAN PATENT APPLICATION

(11) **EP 4 318 372 A1**
(43) Date of publication of application: **07.02.2024**
(21) Application number: 21932854.9
(22) Date of filing: 22.03.2021
(51) Int. Cl.: G06T 1/00, A61B 5/1171

(54) **IRIS IMAGING SYSTEM, IRIS IMAGING METHOD, AND COMPUTER PROGRAM**

(71) Applicant: NEC Corporation, 108-8001 Tokyo (JP)
(72) Inventor: OAMI, Ryoma, Tokyo 108-8001 (JP)
(74) Representative: Betten & Resch
(86) International application number: PCT/JP2021/011698
(87) International publication number: WO 2022/201240

(57) **Abstract**

An iris capturing system (10) comprises: a capturing means (110) for capturing an image including an iris of a living body; an illuminance acquiring means (120) for acquiring brightness when capturing the image; and an execution means (130) for executing, on the basis of brightness when capturing the image, control for bringing a distance between the iris of the living body and the capturing means closer when capturing the image. According to such the iris capturing system, it is possible to appropriately capture images of an iris even if a size of the iris is changing.

## Description

### Technical Field

This disclosure relates to the art of an iris capturing system, an iris capturing method and a computer program for capturing an iris of a living body.

### Background Art

As a system of this type, for example, one that captures an image of an iris of a living body in order to perform iris authentication is known. For example, Patent Literature 1 discloses a technique for adjusting a brightness value of a display according to the pupil size. Patent Literature 2 discloses a technique for guiding a user to an appropriate position when capturing an iris image. Patent Literature 3 discloses a technique of performing high resolution processing on iris images. Patent Literature 4 discloses a technique for completing control relating to focusing and exposure in the interior of a solid-state capturing apparatus. In Patent Literature 5, it is disclosed that an image size and a focus are changed by an individual iris structure.

### Citation List

### Patent Literature

Patent Literature 1: Japanese Patent Application Laid Open No. 2018-073369
Patent Literature 2: International Publication No. 2009/016846
Patent Literature 3: Japanese Patent Application Laid Open No. 2020-071627
Patent Literature 4: Japanese Patent Application Laid Open No. 2018-185749
Patent Literature 5: Japanese Patent Application Laid Open No. 2017-502366

### Summary

### Technical Problem

This disclosure has been made, for example, in view of the above-described respective cited documents, and an object thereof is to provide an iris capturing system, an iris capturing method and a computer program capable of appropriately capturing an image of the iris.

### Solution to Problem

An aspect of an iris capturing system of this disclosure comprises: a capturing means for capturing an image including an iris of a living body; an illuminance acquiring means for acquiring brightness when capturing the image; and an execution means for executing, on the basis of the brightness when capturing the image, control for bringing a distance between the iris of the living body and the capturing means closer when capturing the image.

Another aspect of the iris capturing system of this disclosure comprises: a capturing means for capturing an image including an iris of a living body; a pupil diameter acquiring means for acquiring information about a pupil diameter of the living body; and an execution means for executing, on the basis of the information about the pupil diameter of the living body, control for bringing a distance between the iris of the living body and the capturing means closer when capturing the image.

An aspect of an iris capturing method of this disclosure is an iris capturing method using a capturing means for capturing an image including an iris of a living body, the iris capturing method comprising: acquiring brightness when capturing the image; and performing, on the basis of the brightness when capturing the image, control for bringing a distance between the iris of the living body and the capturing means closer when capturing the image.

An aspect of a computer program of this disclosure causes a computer to execute an iris capturing method using an iris capturing means for capturing an image including an iris of a living body, the iris capturing method comprising: acquiring brightness when capturing the image and performing, on the basis of the brightness when capturing the image, control for bringing a distance between the iris of the living body and the capturing means closer when capturing the image.

### Brief Description of Drawings

[FIG. 1] FIG. 1 is a block diagram showing a hardware configuration of an iris capturing system according to a first embodiment.
[FIG. 2] FIG. 2 is a block diagram showing a functional configuration of the iris capturing system according to the first embodiment.
[FIG. 3] FIG. 3 is a conceptual diagram showing an example in which a pupil is too small, and an example in which a pupil is too large.
[FIG. 4] FIG. 4 is a flowchart showing flow of operation of the iris capturing system according to the first embodiment.
[FIG. 5] FIG. 5 is a block diagram showing a functional configuration of an iris capturing system according to a modification of the first embodiment.
[FIG. 6] FIG. 6 is a flowchart showing the flow of operation of the iris capturing system according to a modification of the first embodiment.
[FIG. 7] FIG. 7 is a graph (part 1) showing a relationship between brightness and a capturing distance.
[FIG. 8] FIG. 8 is a graph (part 2) showing the relationship between brightness and the capturing distance.
[FIG. 9] FIG. 9 is a graph (part 3) showing the relationship between brightness and the capturing distance.
[FIG. 10] FIG. 10 is a graph (part 4) showing the relationship between brightness and the capturing distance.
[FIG. 11] FIG. 11 is a block diagram showing a functional configuration of an iris capturing system according to a third embodiment.
[FIG. 12] FIG. 12 is a flowchart showing flow of operation of the iris capturing system according to the third embodiment.
[FIG. 13] FIG. 13 is a block diagram showing a functional configuration of an iris capturing system according to a fourth embodiment.
[FIG. 14] FIG. 14 is a flowchart showing flow of operation of the iris capturing system according to the fourth embodiment.
[FIG. 15] FIG. 15 is a block diagram showing a functional configuration of an iris capturing system according to a modification of the fourth embodiment.
[FIG. 16] FIG. 16 is a block diagram showing a functional configuration of an iris capturing system according to a fifth embodiment.
[FIG. 17] FIG. 17 is a conceptual diagram (part 1) showing a display example when guiding a standing position.
[FIG. 18] FIG. 18 is a conceptual diagram (part 2) showing a display example when guiding the standing position.
[FIG. 19] FIG. 19 is a flowchart showing flow of operation of the iris capturing system according to the fifth embodiment.
[FIG. 20] FIG. 20 is a block diagram showing a functional configuration of an iris capturing system according to a sixth embodiment.
[FIG. 21] FIG. 21 is a conceptual diagram showing an example of a focus position before and after change.
[FIG. 22] FIG. 22 is a flowchart showing flow of operation of the iris capturing system according to the sixth embodiment.
[FIG. 23] FIG. 23 is a block diagram showing a functional configuration of an iris capturing system according to a seventh embodiment.
[FIG. 24] FIG. 24 is a flowchart showing a flow of the operation of the iris capturing system according to the seventh embodiment.
[FIG. 25] FIG. 25 is a block diagram showing a functional configuration of an iris capturing system according to an eighth embodiment.
[FIG. 26] FIG. 26 is a flowchart showing flow of operation of the iris capturing system according to the eighth embodiment.
[FIG. 27] FIG. 27 is a block diagram showing a functional configuration of an iris capturing system according to a modification of the eighth embodiment.
[FIG. 28] FIG. 28 is a conceptual diagram showing an example of changing a focus position by the iris capturing system according to the modification of the eighth embodiment.

### Description of Embodiments

Hereinafter, embodiments of an iris capturing system, an iris capturing method and a computer program will be described referring to drawings.

### <First embodiment>

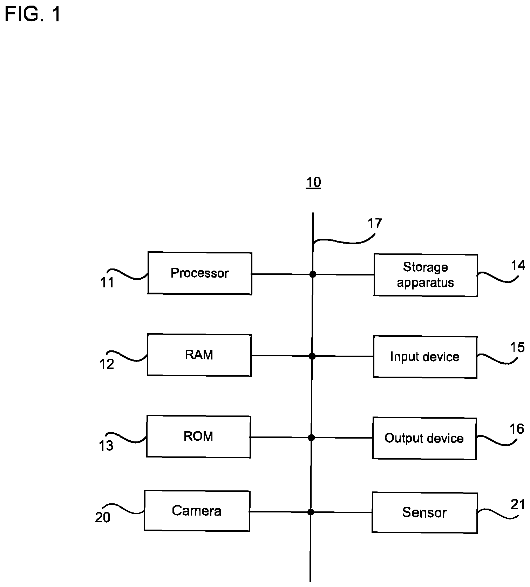

An iris capturing system according to a first embodiment will be described referring to FIGS. 1 to 6.

### (Hardware configuration)

First, a hardware configuration of an iris capturing system 10 according to the first embodiment will be described referring to FIG. 1. FIG. 1 is a block diagram showing a hardware configuration of the iris capturing system according to the first embodiment.

As shown in FIG. 1, the iris capturing system 10 according to the first embodiment includes a processor 11, a RAM (Random Access Memory) 12, a ROM (Read Only Memory) 13 and a storage apparatus 14. The iris capturing system 10 may also include an input device 15 and an output device 16. The iris capturing system 10 may further include a camera 20 and a sensor 21. The processor 11, the RAM 12, the ROM 13, the storage apparatus 14, the input device 15, the output device 16, the camera 20 and the sensor 21 are connected via a data bus 17.

The processor 11 reads computer programs. For example, the processor 11 is configured to read a computer program stored in at least one of the RAM 12, the ROM 13 and the storage apparatus 14. Alternatively, the processor 11 may read a computer program stored in a computer-readable recording medium using a recording medium reading apparatus (not shown). The processor 11 may acquire (i.e., read) a computer program from an apparatus (not shown) located external to the iris capturing system 10 via a network interface. The processor 11 controls the RAM 12, the storage apparatus 14, the input device 15 and the output device 16 by executing the read computer program. In particular, in the present embodiment, when a computer program read by the processor 11 is executed, a functional block for executing a processing for capturing an image of an iris is implemented in the processor 11. As the processor 11, one of the CPU (Central Processing Unit), GPU (Graphics Processing Unit), FPGA (field-programmable gate array), DSP (Demand-Side Platform) and ASIC (Application Specific Integrated Circuit) may be used, or a plurality may be used in parallel.

The RAM 12 temporarily stores computer programs executed by the processor 11. The RAM 12 temporarily stores data for use by the processor 11 when the processor 11 is executing a computer program. The RAM 12 may be, for example, D-RAM (Dynamic RAM).

The ROM 13 stores computer programs executed by the processor 11. The ROM 13 may store other fixed data. The ROM 13 may be, for example, P-ROM (Programmable ROM).

The storage apparatus 14 stores data that the iris capturing system 10 stores over time. The storage apparatus 14 may operate as a temporary storage apparatus of the processor 11. The storage apparatus 14 may include, for example, at least one of a hard disk apparatus, a magnetooptical disk apparatus, an SSD (Solid State Drive) and a disk array apparatus.

The input device 15 is a device that receives input instructions from a user of the iris capturing system 10. The input device 15 may include, for example, at least one of a keyboard, a mouse and a touch panel.

The output device 16 is a device that outputs information about the iris capturing system 10 to the outside. For example, the output device 16 may be a display apparatus (e.g., a display) capable of displaying information about the iris capturing system 10.

The camera 20 is a camera capable of capturing an iris of a living body. The camera 20 may be configured, for example, as a near-infrared camera. The camera 20 may be arranged at a position such that a face and a periocular region of the living body is included in a capturing range. The camera 20 may be a camera for capturing a still image or a camera for capturing a video.

The sensor 21 is a sensor capable of detecting illuminance around the camera. The sensor 21 is configured to output information about the detected illuminance. The sensor 21 may be a sensor capable of detecting illuminance directly, or may be a sensor capable of detecting information different from the illuminance and indirectly detecting illuminance from the information.

### (Functional configuration)

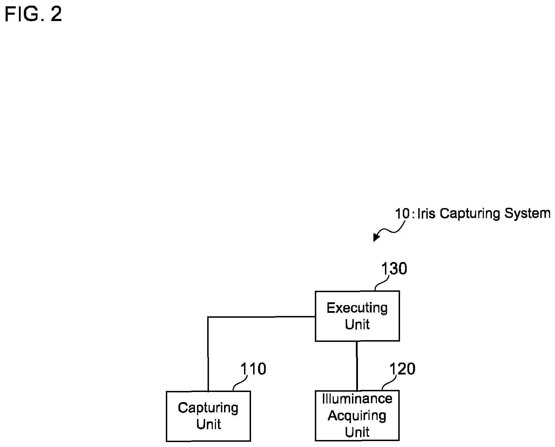

Next, a functional configuration of the iris capturing system 10 according to the first embodiment will be described referring to FIG. 2. FIG. 2 is a block diagram showing a functional configuration of the iris capturing system according to the first embodiment.

As shown in FIG. 2, the iris capturing system 10 according to the first embodiment is configured to include a capturing unit 110, an illuminance acquiring unit 120 and an executing unit 130 as processing blocks for realizing the functions. The capturing unit 110 may be configured to include, for example, a camera 20 described above (see FIG. 1). The illuminance acquiring unit 120 may be configured to include, for example, the above-described sensor 21 (see FIG. 1). The executing unit 130 may be implemented, for example, in the processor 11 described above (see FIG. 1).

The capturing unit 110 is configured to be capable of capturing an iris of a living body. The capturing unit 110 may have a function of outputting an image of the iris of the captured living body (hereinafter, appropriately referred to as "iris image") to the outside of the system. The iris image captured by the capturing unit 110 may be used for iris authentication, for example.

The illuminance acquiring unit 120 is configured to be capable of acquiring brightness (i.e., illuminance) when the capturing unit 110 captures an iris image. The illuminance acquiring unit 120 may acquire information relating to brightness directly from, for example, an illuminance sensor or the like, or may acquire information relating to brightness indirectly from information not directly related to the illuminance. The illuminance acquiring unit 120 may acquire information relating to brightness from, for example, time information. In this case, the illuminance acquiring unit 120 may acquire information on the brightness using a map or the like indicating a relationship between time and brightness prepared in advance.

The executing unit 130 is configured to be capable of executing control to bring a distance between an iris of a living body and the capturing unit 110 (hereinafter, appropriately, "capturing distance") close to each other when the capturing unit 110 captures the iris image on the basis of the information relating to the brightness acquired by the illuminance acquiring unit 120. The executing unit 130 may determine whether or not executing control on the basis of, for example, brightness, or the executing unit 130 may change control amount and control contents in the control. Specific contents of the control executed by the executing unit 130 will be described in detail in other embodiments to be described later.

### (Iris change)

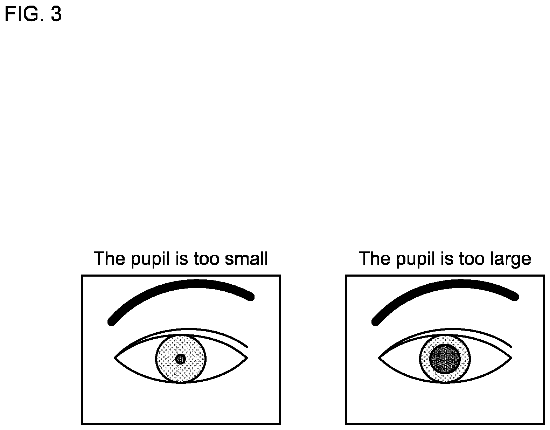

Next, referring to FIG. 3, variations in an iris depending on environment (particularly, changes in a size of a pupil) will be concretely described. FIG. 3 is a conceptual diagram showing an example in which a pupil is too small, and an example in which a pupil is too large.

As shown in FIG. 3, the size of a pupil of a living body varies according to brightness. Specifically, in a bright environment, the pupil becomes small. On the other hand, in a dark environment, the pupil becomes large. This change in pupil size is accompanied by a change in iris status. Therefore, a variation in the size of the pupil considerably affects the iris image captured by the capturing unit 110. As a result, the iris image cannot be obtained in an appropriate state, which may lead to unexpected inconvenience.

For example, when iris images are used for iris authentication, there is a possibility that normal authentication operation cannot be performed when an iris state changes. Specifically, if the pupil becomes too small, the iris pattern located just outside the pupil shrinks, reducing the circumferential resolution of the pupil. On the other hand, if the pupil becomes too large, the entire iris is compressed in a radial direction of the pupil, reducing the radial resolution. The iris capturing system 10 according to this embodiment aims to acquire an appropriate iris image (specifically, an iris image with sufficient resolution) even when the size of the pupil changes as described above.

### (Flow of operation)

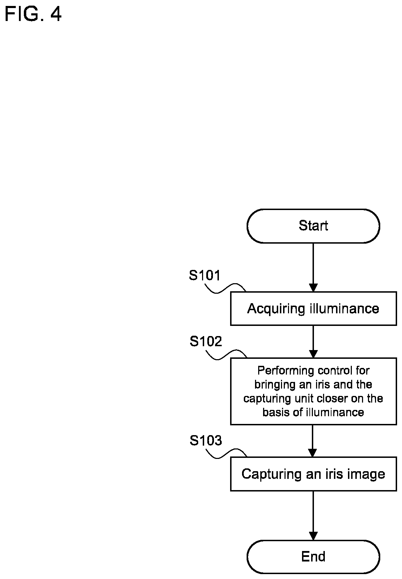

Next, flow of operation of the iris capturing system 10 according to the first embodiment will be described referring to FIG. 4. FIG. 4 is a flowchart showing the flow of the operation of the iris capturing system according to the first embodiment.

As shown in FIG. 4, when the iris capturing system 10 according to the first embodiment operates, first, the illuminance acquiring unit 120 acquires illuminance of a periphery of the capturing unit 110 (stepnS 101). The illuminance acquiring unit 120 outputs the acquired illuminance to the executing unit 130.

Subsequently, the executing unit 130 executes control to bring the capturing distance closer on the basis of the illuminance acquired by the illuminance acquiring unit 120 (step S102). Then, the capturing unit 110 captures the iris image of the living body after the executing unit 130 executes the control of bringing the capturing distance closer (step S103). Therefore, the iris image is captured in a condition where the capturing distance is close in accordance with the illuminance.

In the case where it is determined that the executing unit 130 does not need to execute the control for bringing the capturing distance closer (the case where the illuminance is within a range in which bringing the capturing distance closer is unnecessary), the capturing unit 110 may capture the iris image in a condition in which the control by the executing unit 130 is not executed.

### (Modification)

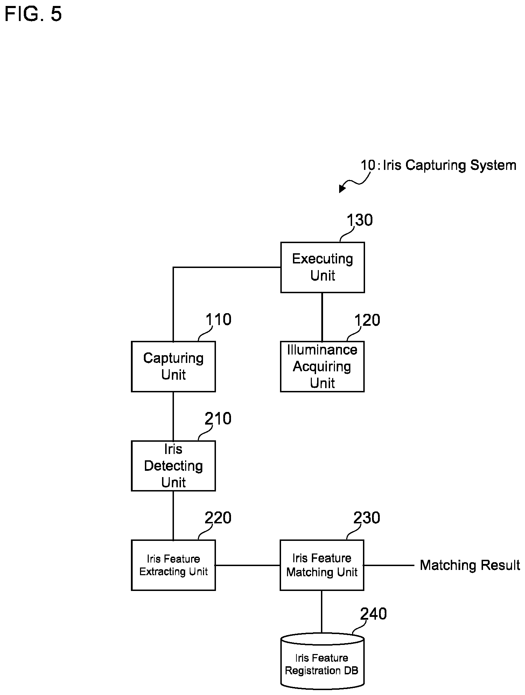

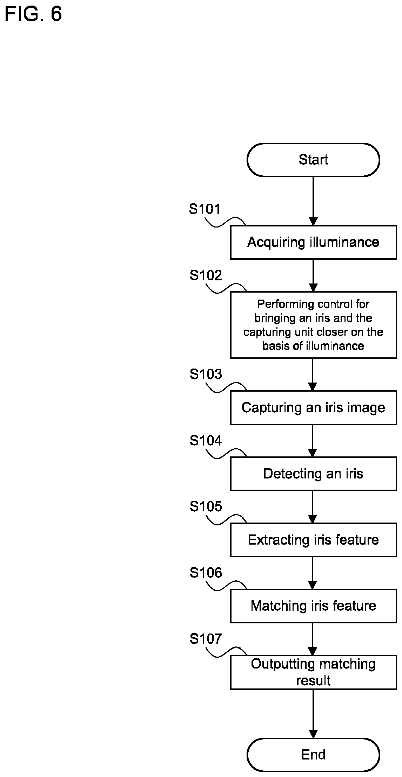

Next, an iris capturing system 10 according to a modification of the first embodiment will be described referring to FIGS. 5 and 6. FIG. 5 is a block diagram showing a functional configuration of the iris capturing system according to the modification of the first embodiment. In FIG. 5, the same reference numerals denote the same elements as the components shown in FIG. 2. FIG. 6 is a flowchart showing flow of operation of the iris capturing system according to the modification of the first embodiment. In FIG. 6, processing similar to that shown in FIG. 4 is denoted by the same reference numerals.

Incidentally, the modification described below only differs in some configurations and operations as compared with the first embodiment, the other portions may be the same as the first embodiment (see FIGS. 1 to 4). Therefore, the portions that differ from the first embodiment described above will be described in detail below, and other overlapping portions will not be described as appropriate.

As shown in FIG. 5, the iris capturing system 10 according to the modification of the first embodiment includes a capturing unit 110, an illuminance acquiring unit 120, an executing unit 130, an iris detecting unit 210, an iris feature extracting unit 220, an iris feature matching unit 230 and an iris feature registering database 240 as processing blocks for realizing the functions. That is, the iris capturing system 10 according to the modified example is further configured to include the iris detection unit 210, the iris feature extracting unit 220, the iris feature matching unit 230 and the iris feature registration database 240 in addition to the configuration of the iris capturing system 10 according to the first embodiment (see FIG. 2). Note that each of the iris detection unit 210, the iris feature extraction unit 220 and the iris feature matching unit 230 may be realized in, for example, the above-described processor 11 (see FIG. 1). The iris feature registration database 240 may be configured to include the storage apparatus 14 (see FIG. 1) described above.

The iris detecting unit 210 is configured to be capable of detecting area (in other words, the position of the iris in the image) in which the iris exists from the iris image. The iris detecting unit 210 is configured to output information relating to the detected position of the iris to the iris feature extracting unit 220.

The iris feature extracting unit 220 is configured to be capable of extracting the feature of the iris from area in which the iris detected by the iris detecting unit 210 is present. The feature here is the information used for iris recognition (i.e., verification with registered data processing). The iris feature extracting unit 220 may be made to extract two or more kinds of features. The feature extracted by the iris feature extracting unit 220 is configured to be output to the iris feature matching unit 230.

The iris feature matching unit 230 is configured to output the matching result relating to the iris by matching the feature (hereinafter, appropriately referred to as "extracted feature") extracted by the iris feature extracting unit 220 with the feature (hereinafter, appropriately referred to as "registered feature") registered in the iris feature registration database 240 to be described later. The iris feature matching unit 230 outputs the matching result on the basis of, for example, the degree of coincidence between the extracted feature and the registered feature. For example, the iris feature matching unit 230 may output a result of matching OK when the degree of coincidence between the extracted feature and the registered feature is equal to or greater than a predetermined threshold, and may output a result of matching NG when the degree of coincidence is less than the predetermined value.

The iris feature registration database 240 is configured to store the registered feature used for verification by the iris feature matching unit 230. The registered feature may be data registered in advance. The registered feature stored in the iris feature registration database 240 is configured to be appropriately read by the iris feature matching unit 230.

As illustrated in FIG. 6, when the iris capturing system 10 according to a modification of the first embodiment operates, first, the illuminance acquiring unit 120 acquires the illuminance around the capturing unit 110 (step S101). The illuminance acquiring unit 120 outputs the acquired illuminance to the executing unit 130.

Subsequently, the executing unit 130 executes control to bring the capturing distance closer on the basis of the illuminance acquired by the illuminance acquiring unit 120 (step S102). Then, the capturing unit 110 captures the iris image of the living body after the executing unit 130 executes the control of bringing the capturing distance closer (step S103).

Subsequently, the iris detection unit 210 detects area in which the iris exists from the iris image (step S104). Then, the iris feature extracting unit 220 extracts the feature of the iris from area in which the iris is present (step S 105).

Subsequently, the iris feature matching unit 230 matches the extracted feature extracted by the iris feature extracting unit 220 with the registered feature stored in the iris feature registration database 240 (step S106). The iris feature matching unit 230 outputs the matching result (step S107).

### (Technical effects)

Next, technical effects obtained by the iris capturing system 10 according to the first embodiment will be described.

As described referring to FIGS. 1 to 6, in the iris capturing system 10 according to the first embodiment, control is performed to bring the capturing distance (that is, the distance between the iris of the living body and the capturing unit 110) close to each other on the basis of the illuminance (brightness) when the iris image is captured. As the capturing distance approaches, the iris will be imaged closer. Therefore, the resolution of the iris image is higher than before the capturing distance is close. Therefore, according to the iris capturing system 10 according to the first embodiment, it is possible to capture an iris image having a high resolution in consideration of the size of the pupil that varies according to the brightness. In particular, in the configuration for executing iris authentication (that is, matching the feature of the iris) described in the modified example, in many cases, a high-resolution iris image is required to accurately extract the feature. Therefore, the above-described technical effects are significantly exhibited when performing iris recognition.

### <Second embodiment>

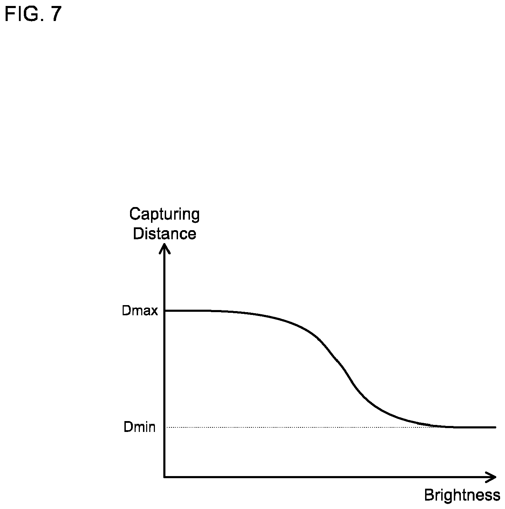

An iris capturing system 10 according to a second embodiment will be described referring to FIGS. 7 to 10. The second embodiment explains that the control performed in the first embodiment described above (i.e., a specific example of the control executed by the executing unit 130), a configuration of a system and flow of operation of the second embodiment may be the same as the first embodiment (see FIGS 1 to 6), for example. Therefore, in the following, the description of the portion overlapping with the first embodiment already described is omitted.

### (Setting method of capturing distance)

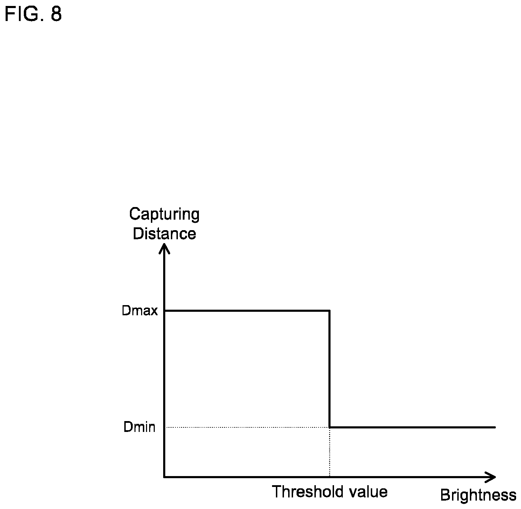

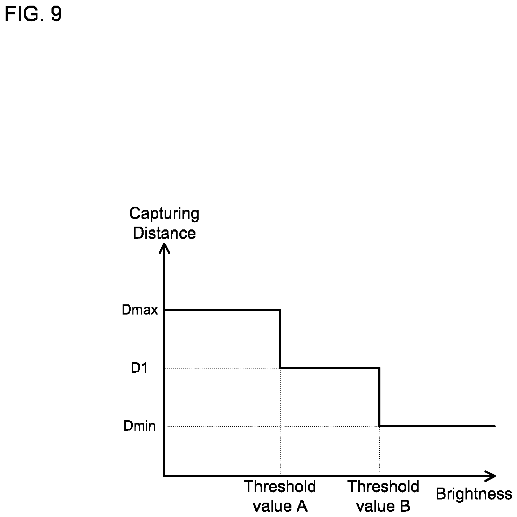

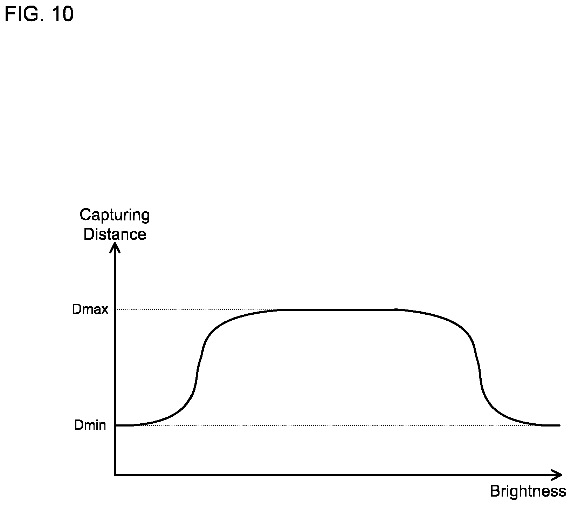

First, a setting method of a capturing distance in the iris capturing system 10 according to the second embodiment will be specifically described referring to FIGS. 7 to 10. FIG. 7 is a graph (part 1) showing a relationship between brightness and the capturing distance. FIG. 8 is a graph (part 2) showing the relationship between brightness and the capturing distance. FIG. 9 is a graph (part 3) showing the relationship between brightness and the capturing distance. FIG. 10 is a graph (part 4) showing the relationship between brightness and the capturing distance.

As shown in FIG. 7, the executing unit 130 may perform control so that the capturing distance (that is, the distance between the iris of the living body and the capturing unit 110) becomes closer as the environment when capturing the iris image becomes brighter. Incidentally, the executing unit 130 may perform control so as to change the capturing range within a predetermined range. For example, in the exemplary embodiment shown in FIG. 7, the capturing distance is controlled within range of Dmax - Dmin.

As shown in FIG. 8, the executing unit 130 may perform control so as to change the capturing distance at a stage in which the brightness when capturing the iris image exceeds a predetermined threshold value. For example, in the example shown in FIG. 8, when the brightness is less than the predetermined threshold, the capturing distance is controlled to be Dmax, and when the brightness is equal to or greater than the predetermined threshold, the capturing distance is controlled to be Dmin.

As shown in FIG. 9, the executing unit 130, using two or more thresholds, may be controlled to change the capturing distance. For example, in the example shown in FIG. 9, when the brightness is less than a threshold value A, the capturing distance is controlled so that the capturing distance is Dmax, when the brightness is greater than or equal to the threshold value A, and when the brightness is less than a threshold value B, the capturing distance is controlled so that the capturing distance is D1, and when the brightness is greater than or equal to the threshold value B, the capturing distance is controlled so that the capturing distance is Dmin.

As shown in FIG. 10, the executing unit 130 may perform control to bring the capturing distance closer not only when the environment when capturing the iris image becomes brighter but also when it becomes darker. As already described, the size of the pupil is not only too small for bright cases, but also too large for dark cases (see FIG. 3). Therefore, in a situation where the size of the pupil is assumed to be too large due to a dark environment in which an iris image is captured, the control of bringing the capturing distance closer may be performed in the same manner.

Incidentally, up to this point, the control of bringing the capturing distance closer to one target distance has been described, the target distance may have a certain width. For example, the control may be performed so as to fit a distance within a fixed range before and after the target capturing distance described above.

### (Technical effects)

Next, technical effects obtained by the iris capturing system 10 according to the second embodiment will be described.

As described referring to FIGS. 7 to 10, in the iris capturing system 10 according to the second embodiment, the brighter the environment when capturing an image is, the closer the capturing distance. In this way, it is possible to capture an iris image with high resolution in consideration of variations in the size of the pupil according to the brightness. Similarly, if the same control is performed in the dark case, it is possible to capture an iris image with a high resolution by considering the variation of the pupil size according to the darkness.

### <Third embodiment>

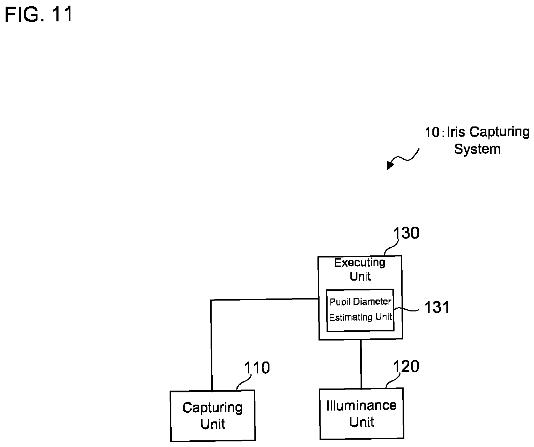

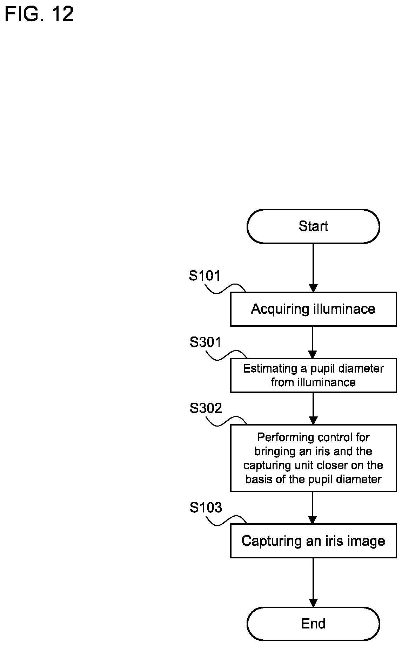

An iris capturing system 10 according to a third embodiment will be described referring to FIGS. 11 and 12. Incidentally, the third embodiment only differs in some configurations and operations as compared with the first and second embodiments described above, the other portions may be the same as the first and second embodiments. Therefore, in the following, it is intended to omit the appropriate description for portions overlapping with the embodiments already described.

### (Functional configuration)

First, a functional configuration of the iris capturing system 10 according to the third embodiment will be described referring to FIG. 11. FIG. 11 is a block diagram showing the functional configuration of the iris capturing system according to the third embodiment. Incidentally, in FIG. 11, it is denoted by the same reference numerals to the same elements as the components shown in FIG. 2.

As shown in FIG. 11, the iris capturing system 10 according to the third embodiment is configured to include a capturing unit 110, an illuminance acquiring unit 120 and an executing unit 130 as processing blocks for realizing the functions. In particular, the iris capturing system 10 according to the third embodiment, the executing unit 130 includes a pupil diameter estimating unit 131.

The pupil diameter estimating unit 131 is configured to be able to estimate the pupil diameter of the living body from the brightness when capturing the image. The size of the pupil of a living body changes according to the brightness of the environment, as already described (see FIG. 3). Therefore, if information on brightness is used, it is possible to estimate the pupil diameter of the living body at that time. Incidentally, the pupil diameter estimating unit 131, in addition to the information on brightness, may be configured to be able to estimate the pupil diameter using time information or the like. Information of the pupil diameter estimated by the pupil diameter estimating unit 131 is used for the control executed by the control executing unit 130. That is, in the third embodiment, on the basis of the pupil diameter estimated from the brightness, the control of bringing the capturing distance closer is executed. For example, when environment is bright, the capturing distance is controlled to be close to a capturing distance that is obtained as a distance at which the number of pixels around a circumference of a pupil is equal to or greater than a certain value, and an entire iris is captured with an appropriate size. On the other hand, when environment is dark, the capturing distance is controlled to be close to a capturing distance that is obtained as a distance at which the number of pixels in a radial direction between an iris and a pupil is equal to or greater than a certain value, and an entire iris is imaged with an appropriate size. Here, while the resolution of the iris increases and the pattern of the iris becomes clear when the iris is imaged larger, if the position of the iris deviates even a little, it may protrude out of the image, resulting in a possibility that the entire image cannot be imaged. Also, if you are too close, the capturing distance may exceed a focusing capable range. For this reason, the appropriate size of the iris is determined by considering these trade-offs, and the capturing distance is determined.

### (Flow of operation)

Next, flow of operation of the iris capturing system 10 according to the third embodiment will be described referring to FIG. 12. FIG. 12 is a flowchart showing the flow of the operation of the iris capturing system according to the third embodiment. Incidentally, in FIG. 12, the same reference numerals are given to the same processing as shown in FIG. 4.

As shown in FIG. 12, when the iris capturing system 10 according to the third embodiment operates, first, the illuminance acquiring unit 120 acquires the illuminance around the capturing unit 110 (step S101). The illuminance acquiring unit 120 outputs the acquired illuminance to the executing unit 130.

Subsequently, the pupil diameter estimating unit 131 in the executing unit 130 estimates the pupil diameter of the living body from the illuminance acquired by the illuminance acquiring unit 120 (step S301). Then, the executing unit 130 on the basis of the pupil diameter estimated by the pupil diameter estimating unit 131 executes a control to close the capturing distance (step S302).

Thereafter, the capturing unit 110 captures the iris image of the living body after the executing unit 130 executes the control of bringing the capturing distance closer (step S103). When it is determined that the executing unit 130 does not need to perform the control for bringing the capturing distance closer (when the pupil diameter is within a range in which bringing the capturing distance closer is unnecessary), the capturing unit 110 may capture the iris image in a condition in which the control by the executing unit 130 is not executed.

### (Technical effects)

Next, technical effects obtained by the iris capturing system 10 according to the third embodiment will be described.

As described referring to FIGS. 11 and 12, in the iris capturing system 10 according to the third embodiment, the pupil diameter is estimated from the brightness when capturing the image, and control for bringing the capturing distance closer is performed on the basis of the pupil diameter.
In this way, it is possible to capture an iris image with high resolution by more specifically considering variations in the size of the pupil according to the brightness.

### <Fourth embodiment>

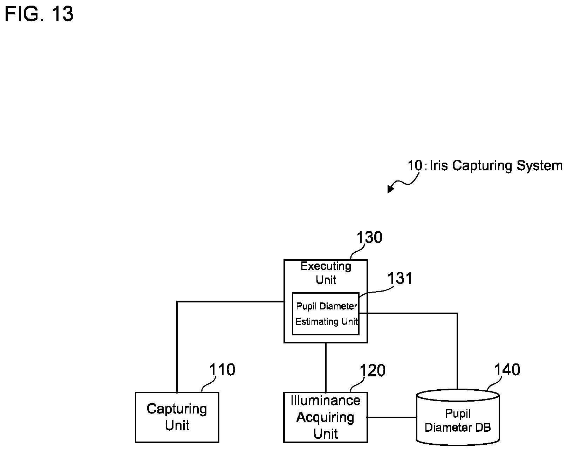

An iris capturing system 10 according to a fourth embodiment will be described referring to FIGS. 13 to 15. Incidentally, the fourth embodiment only differs in some configurations and operations as compared with the third embodiment described above, the other portions may be the same as the first to third embodiments. Accordingly, in the following, the description of the portions overlapping with the embodiments already described will be omitted as appropriate.

### (Functional configuration)

First, a functional configuration of the iris capturing system 10 according to the fourth embodiment will be described referring to FIG. 13. FIG. 13 is a block diagram showing a functional configuration of the iris capturing system according to the fourth embodiment. Incidentally, in FIG. 13, it is denoted by the same reference numerals to the same elements as the components shown in FIG. 11.

As shown in FIG. 13, the iris capturing system 10 according to the fourth embodiment is configured to include a capturing unit 110, an illuminance acquiring unit 120, an executing unit 130 having a pupil diameter estimating unit 131 and a pupil diameter database 140 as processing blocks for realizing the functions. That is, the iris capturing system 10 according to the fourth embodiment further comprises the pupil diameter database 140 in addition to the configuration of the third embodiment (see FIG. 11). The pupil diameter database 140 may be configured to include, for example, the storage apparatus 14 described above (see FIG. 1).

The pupil diameter database 140 is configured to be able to store information indicating a relationship between brightness when capturing the iris image and a pupil diameter of a living body. The pupil diameter database 140 may store, for example, the relationship between the brightness and the pupil diameter as a map, or may store mathematical equations for calculating the pupil diameter from the brightness. Information stored in the pupil diameter database 140 may be one that is prepared in advance (i.e., accumulated by prior experiments and simulations, etc.), may be configured to appropriately update the accumulated information. The pupil diameter database 140 may accumulate the relationship between the brightness and time information, and the pupil diameter.

### (Flow of operation)

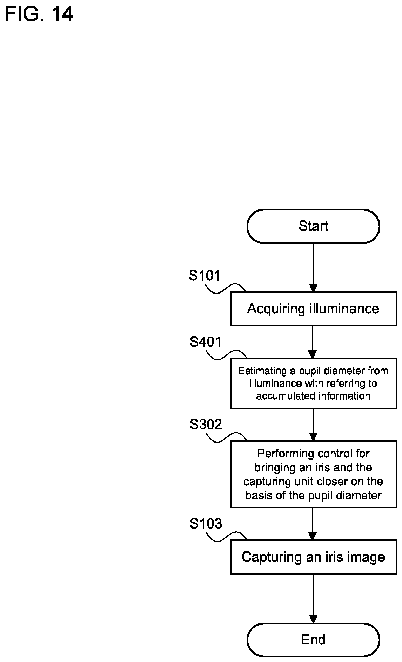

Next, flow of the operation of the iris capturing system 10 according to the fourth embodiment will be described referring to FIG. 14. FIG. 14 is a flowchart showing the flow of the operation of the iris capturing system according to the fourth embodiment. Incidentally, in FIG. 14, the same reference numerals are denoted by the same processing as shown in FIG. 12.

As shown in FIG. 14, when the iris capturing system 10 according to the fourth embodiment operates, first, the illuminance acquiring unit 120 acquires the illuminance around the capturing unit 110 (step S101). The illuminance acquiring unit 120 outputs the acquired illuminance to the executing unit 130.

Subsequently, the pupil diameter estimating unit 131 in the executing unit 130 estimates the pupil diameter of the living body using the illuminance acquired by the illuminance acquiring unit 120 and the information accumulated in the pupil diameter database 140 (step S401). Then, the executing unit 130 on the basis of the pupil diameter estimated by the pupil diameter estimating unit 131 executes a control to close the capturing distance (step S302).

Thereafter, the capturing unit 110 captures the iris image of the living body after the executing unit 130 executes the control of bringing the capturing distance closer (step S103).

### (Configuration example for data accumulation)

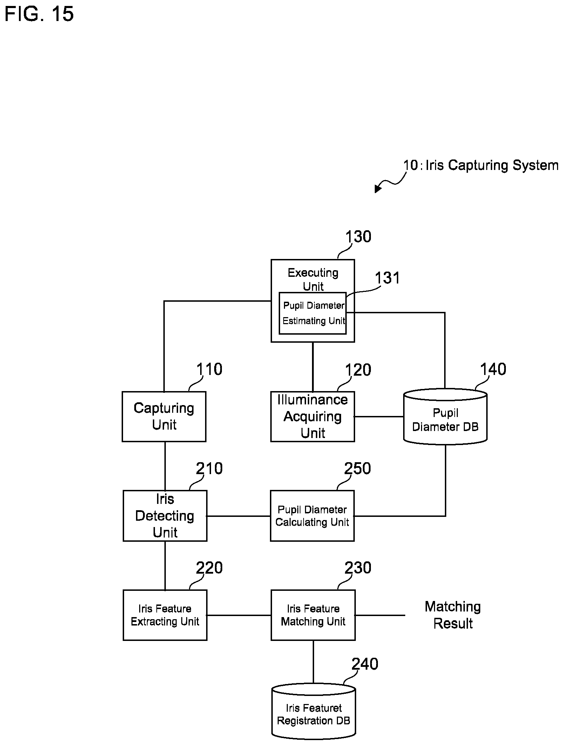

Next, referring to FIG. 15, a configuration example when accumulating data in the pupil diameter database 140 will be described. FIG. 15 is a block diagram showing a functional configuration of an iris capturing system according to a modification of the fourth embodiment. Incidentally, in FIG. 15, it is denoted by the same reference numerals to the same elements as the components shown in FIGS. 5 and 13.

As shown in FIG. 15, the iris capturing system 10 according to the modification of the fourth embodiment includes a capturing unit 110, an illuminance acquiring unit 120, an executing unit 130 having a pupil diameter estimating unit 131, a pupil diameter database 140, an iris detecting unit 210, an iris feature extracting unit 220, an iris feature matching unit 230, an iris feature registering database 240 and a pupil diameter calculating unit 250 as processing blocks for realizing the functions. That is, the iris capturing system 10 according to the modification of the fourth embodiment further includes the iris detection unit 210, the iris feature extracting unit 220, the iris feature matching unit 230, the iris feature registering database 240 and the pupil diameter calculating unit 250 in addition to the configuration (see FIG. 13) of the fourth embodiment. Each of the iris detection unit 210, the iris feature extracting unit 220, the iris feature matching unit 230 and the iris feature registration database 240 may be the same as that described in the modified example (see FIG. 5) of the first embodiment. Further, the pupil diameter calculation unit 250 may be implemented in, for example, the above-described processor 11 (see FIG. 1).

The pupil diameter calculating unit 250 is configured to be capable of calculating the pupil diameter from area in which the pupil detected by the iris detecting unit 210 exists. Incidentally, as for the specific method of calculating the pupil diameter from the image, it is possible to appropriately adopt the existing technique, a detailed description thereof will be omitted. The pupil diameter calculating unit 250 is configured to appropriately store information on the calculated pupil diameter in the pupil diameter database 140. Therefore, each time the pupil diameter is calculated by the pupil diameter calculating unit 250 (in other words, each time the iris image is captured by the capturing unit 110), new information is accumulated in the pupil diameter database 140. Thus, the pupil diameter database 140, using the capturing result of the capturing unit 110, may accumulate new information about the pupil diameter. However, the pupil diameter database 140 may store information other than the pupil diameter calculated by the pupil diameter calculating unit 250. Then, when information is accumulated to some extent, statistical processing may be performed on the newly accumulated information to update the map or mathematical expression representing the relation between the brightness and the pupil diameter. For example, the pupil diameter statistics (representative values such as the mean value, median value, and mode value) may be calculated for each illuminance, and the map for obtaining the pupil diameter from the illuminance may be updated. At this time, a correspondence relationship may be calculated not only by using the newly added value, but also by considering the information already accumulated. In addition, if the pupil diameter is accumulated in association with the time data, a similar statistical processing may be performed for each time of day. In this case, the relationship between the brightness and the time of day may be used to obtain a map or mathematical formula for obtaining the pupil diameter from the information of the time of day instead of the brightness.

### (Technical effects)

Next, technical effects obtained by the iris capturing system 10 according to the fourth embodiment will be described.

As described referring to FIGS. 13 to 15, in the iris capturing system 10 according to the fourth embodiment, the pupil diameter is estimated using the information accumulated in the pupil diameter database 140. In this way, since it is possible to accurately estimate the pupil diameter using the accumulated information, it is possible to more appropriately perform the control of bringing capturing distance closer.

### <Fifth embodiment>

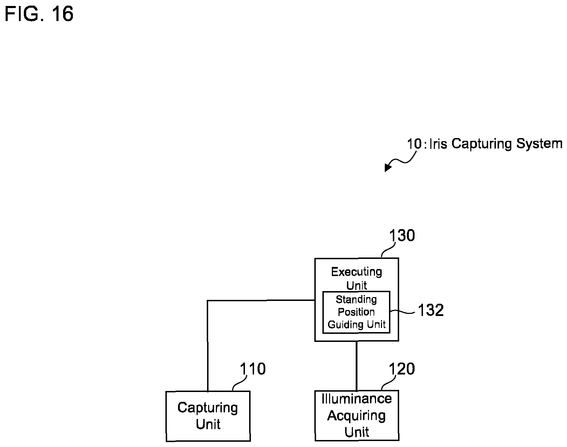

An iris capturing system 10 according to a fifth embodiment will be described referring to FIGS. 16 to 19. Incidentally, the fifth embodiment only differs in some configurations and operations as compared with the first to fourth embodiments described above, the other portions may be the same as the first to fourth embodiments. Accordingly, in the following, the description of the portions overlapping with the embodiments already described will be omitted as appropriate.

### (Functional configuration)

First, a functional configuration of the iris capturing system 10 according to the fifth embodiment will be described referring to Fig. 16. FIG. 16 is a block diagram showing a functional configuration of the iris capturing system according to the fifth embodiment. Incidentally, in FIG. 16, it is denoted by the same reference numerals to the same elements as the components shown in FIG. 2.

As shown in FIG. 16, the iris capturing system 10 according to the fifth embodiment is configured to include a capturing unit 110, an illuminance acquiring unit 120 and an executing unit 130 as processing blocks for realizing the functions. In particular, the iris capturing system 10 according to the fifth embodiment, the executing unit 130 includes a standing position guiding unit 132.

The standing position guiding unit 132 is configured to be able to notify the living body (that is, the target person that captures the iris image) of information that prompts the movement. Standing position guiding unit 132 may perform a notification prompting to approach the capturing unit 110 to the target person. Incidentally, the standing position guiding unit 132, for example, via the output device 16 (see FIG. 1), may notify the target person. Specifically, the standing position guiding unit 132 may display images or messages that prompt the target person to move. Alternatively, the standing position guiding unit 132 may output, from a microphone, a sound that prompts the target person to move. Specific examples of the guidance method by the standing position guiding unit 132 will be described in more detail below.

### (Display example when guiding)

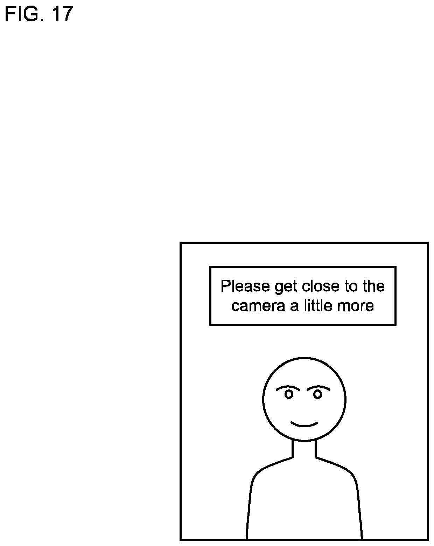

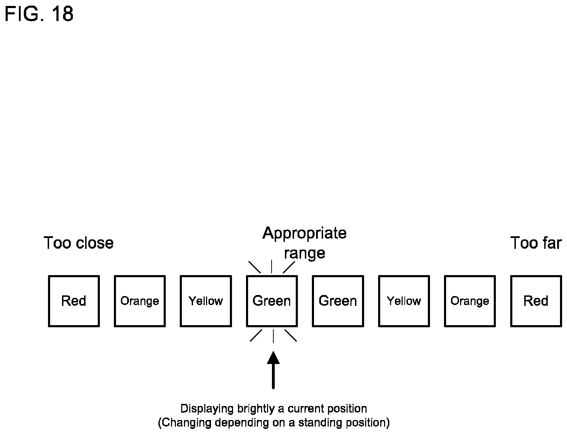

Next, referring to FIGS. 17 and 18, a specific description will be given of a display example when guiding the standing position by the standing position guiding unit 132 described above. FIG. 17 is a conceptual diagram (part 1) showing a display example in guiding the standing position. FIG. 18 is a conceptual diagram (part 2) showing a display example in guiding the standing position.

As shown in FIG. 17, the standing position guiding unit 132 may display a message prompting the target person to move together with an image of the target person. In the example shown in FIG. 17, the message "Please get closer to the camera a little more" is displayed, but it may also include information concretely indicating how close the person need to get. For example, specific amount of movement such as "Please approach 10cm further" may be displayed. Alternatively, a frame indicating the reference position of the target person may be superimposed on the display, and a message such as "Move to fit within the frame" may be displayed. The content of the displayed message may change as the target person moves. For example, if the target person approaches the exact position, a message such as "OK in this position" may be displayed. In addition, if the user gets too close, a message such as "too close. Keep a little further away" may be displayed. Further, a speaker may be connected to announce the message by voice as well as simultaneously displaying the message. Alternatively, the message may not be displayed, but only voice announcements may be made. The distance to the target person may be estimated from the size of the face portion on the image, the positional relationship of the portion, or the size of the iris, or may be measured separately using a distance sensor or the like. The resulting distance may also be used to change the guidance message described above. For example, in a focused range, the appropriate capturing distance may be determined and controlled to approach that distance.

As shown in FIG. 18, the standing position guiding unit 132 may urge the target person to move using an indicator. The indicator includes, for example, red, orange, yellow, green lamps, and a corresponding lamp is configured to be turned on in accordance with the positional relation between the target person and the capturing unit 110. Specifically, when the target person is too close or too far from the capturing unit 110, the red lamp is turned on. Then, as the target person approaches the capturing unit 110, the lamps lighting up change in the order of orange and yellow, and the green lamp is turned on when the distance is just right. Incidentally, the indicator may be configured to guide the standing position by the blinking pattern of the lamp instead of the color of the lamps. For example, if the standing position is apart, the period of blinking becomes faster, and when the standing position approaches the appropriate position, the period of blinking becomes slower, and when the standing position becomes an appropriate position, the blinking may be stopped. The indicator as described above may be configured using an LED lamp, for example, or may be implemented by image display by a display or the like.

Instead of the indicator described above, a contour of the target person may be superimposed on the display and guided so that the target person is just within its contour. In this case, the contour to be superimposed may vary in size according to the brightness when capturing the iris image or the pupil diameter of the present target person. More specifically, when the capturing environment is very bright or very dark, a larger contour may be displayed to make the target person closer, while a slightly larger contour may be displayed to make the target person slightly closer when it is slightly brighter or slightly darker. Similarly, if the pupil diameter of the target person deviates greatly from the appropriate value, a larger contour may be displayed to make the target person closer, while if it deviates slightly, a slightly larger contour may be displayed to make the target person slightly closer.

### (Flow of operation)

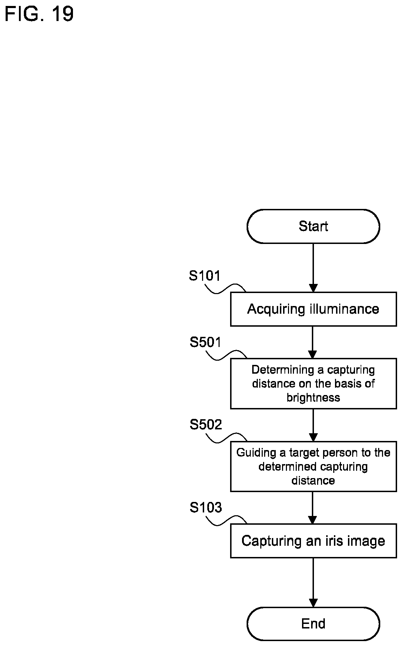

Next, flow of operation of the iris capturing system 10 according to the fifth embodiment will be described referring to FIG. 19. FIG. 19 is a flowchart showing the flow of the operation of the iris capturing system according to the fifth embodiment. Incidentally, in FIG. 19, the same reference numerals are given to the same processing as shown in FIG. 4.

As shown in FIG. 19, when the iris capturing system 10 according to the fifth embodiment operates, first, the illuminance acquiring unit 120 acquires the illuminance around the capturing unit 110 (step S101). The illuminance acquiring unit 120 outputs the acquired illuminance to the executing unit 130.

Subsequently, the executing unit 130 determines an appropriate capturing distance on the basis of the illuminance acquired by the illuminance acquiring unit 120 (step S501). Then, the standing position guiding unit 132, so that the capturing distance becomes the determined capturing distance, performs a standing position guidance to the target person (step S502).

Thereafter, the capturing unit 110 confirms that the target person has moved to an appropriate position by the standing position guiding unit 132, and then captures an iris image of the living body (step S103). Incidentally, if the target person does not move to an appropriate position even though the standing position guidance is performed, the capturing processing may be stopped to notify a system manager or the like.

### (Technical effects)

Next, a technical effect obtained by the iris capturing system 10 according to the fifth embodiment will be described.

As described referring to FIGS. 16 to 19, in the iris capturing system 10 according to the fifth embodiment, control for guiding the standing position of the target person is performed in order to approach the capturing range. In this way, by guiding the standing position of the target person, it is possible to achieve an appropriate capturing range according to the brightness. Configuration of the fifth embodiment is particularly useful, for example, when the target person stops in front of the capturing unit 110 and the capturing is performed.

### <Sixth embodiment>

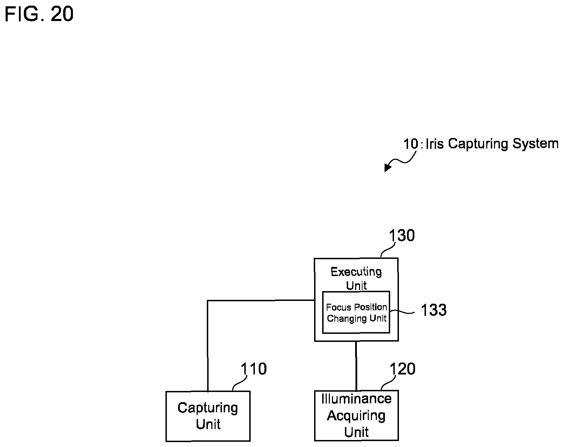

An iris capturing system 10 according to a sixth embodiment will be described referring to FIGS. 20 to 22. Incidentally, the sixth embodiment only differs in some configurations and operations as compared with the first to fifth embodiments described above, the other portions may be the same as the first to fifth embodiments. Accordingly, in the following, the description of the portions overlapping with the embodiments already described will be omitted as appropriate.

### (Functional configuration)

First, a functional configuration of the iris capturing system 10 according to the sixth embodiment will be described referring to FIG. 20. FIG. 20 is a block diagram showing the functional configuration of the iris capturing system according to the sixth embodiment. Incidentally, in FIG. 20, it is denoted by the same reference numerals to the same elements as the components shown in FIG. 2.

As shown in FIG. 20, the iris capturing system 10 according to the sixth embodiment is configured to include a capturing unit 110, an illuminance acquiring unit 120 and an executing unit 130 as processing blocks for realizing the functions. In particular, the iris capturing system 10 according to the sixth embodiment includes the focus position changing unit 133 in the executing unit 130.

The focus position changing unit 133 is configured to be capable of changing the focus position of the capturing unit 110. The focus position changing unit 133 changes the focus position of the capturing unit 110 so as to approach the capturing unit 110 itself. The focus position changing unit 133 may be configured to adjust how close the focus position of the capturing unit 110 is. The focus position changing unit 133, when a plurality of the focus positions of the capturing unit 110 are set, it is sufficient to be configured to change at least one of them. For a specific method of changing the focus position by the focus position changing unit 133 will be described in more detail below.

### (Example of changing the focus position)

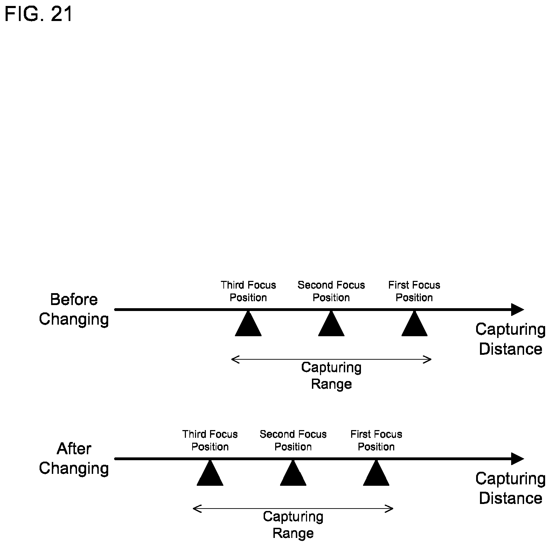

Next, referring to FIG. 21, an example of changing the focus position of the capturing unit 110 by the focus position changing unit 133 described above will be specifically described. FIG. 21 is a conceptual diagram showing an example of the focus position before and after the change.

As shown in FIG. 21, the first, the second, and the third focus positions are set in the capturing unit 110. In this case, the focus position changing unit 133, each of the first, the second, and the third focus positions is changed so that the capturing distance is reduced. The focus position changing unit 133 may change the focus position so that the capturing distance is reduced as the capturing environment becomes brighter. The focus position changing unit 133 may change the focus position so as to fit in the capturing range determined on the basis of the brightness. In the example shown in FIG. 21, the first, the second, and the third focus positions are changed by the same amount collectively, but a plurality of focus positions may be changed independently, respectively.

### (Flow of operation)

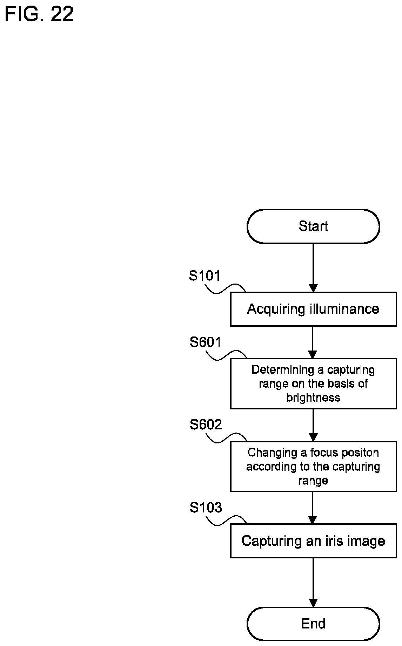

Next, flow of operation of the iris capturing system 10 according to the sixth embodiment will be described referring to FIG. 22. FIG. 22 is a flowchart showing the flow of the operation of the iris capturing system according to the sixth embodiment. Incidentally, in FIG. 22, the same reference numerals are given to the same processing as shown in FIG. 4.

As shown in FIG. 22, when the iris capturing system 10 according to the sixth embodiment operates, first, the illuminance acquiring unit 120 acquires the illuminance around the capturing unit 110 (step S101). The illuminance acquiring unit 120 outputs the acquired illuminance to the executing unit 130.

Subsequently, the executing unit 130 determines the capturing range on the basis of the illuminance acquired by the illuminance acquiring unit 120 (step S601). Then, the focus position changing unit 133 changes the focus position of the capturing unit 110 so as to fit in the determined capturing range (step S602).

Thereafter, the capturing unit 110 captures an iris image of the living body at the focus position changed by the focus position changing unit 133 (step S103). Incidentally, the focus position changing unit 113, in addition to or instead of changing the focus position, may be changed magnification of the capturing unit 110.

### (Technical effects)

Next, a technical effect obtained by the iris capturing system 10 according to the sixth embodiment will be described.

As described in FIGS. 20 to 22, in the iris capturing system 10 according to the sixth embodiment, control is performed to change the focal position of the capturing unit 110 in order to close the capturing distance. In this way, the capturing distance is changed by changing the focal point of the capturing unit 110, it is possible to realize an appropriate capturing distance according to the brightness. The configuration of the sixth embodiment is particularly useful when, for example, the target person is captured a plurality of times while moving in the capturing range of the capturing unit 110. That is, when the target person moves toward the camera, while shifting the focus position in the direction, it is effective when capturing a plurality of times.

### <Seventh embodiment>

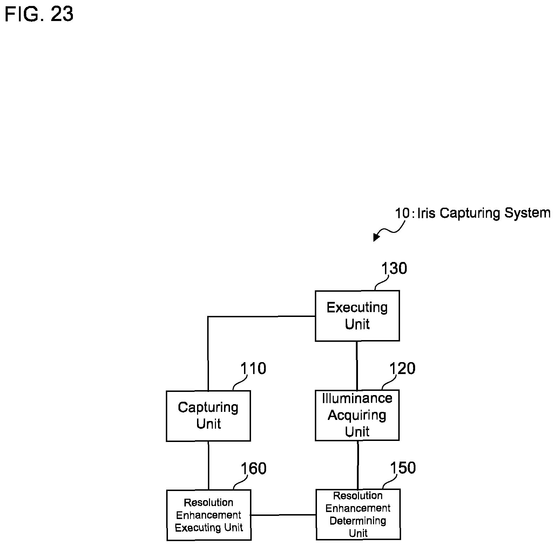

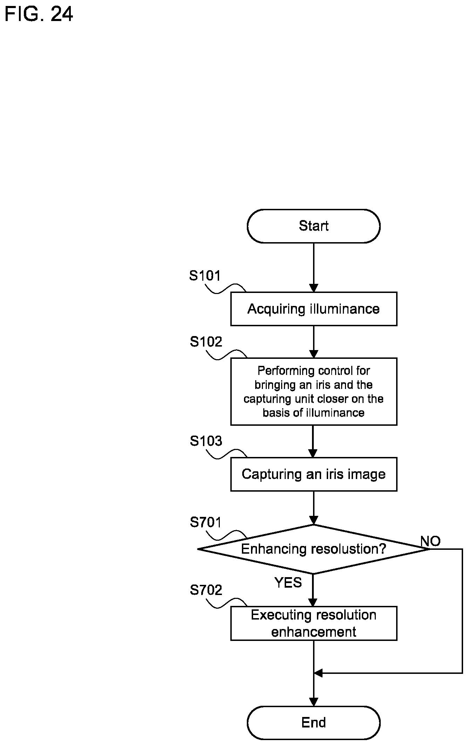

An iris capturing system 10 according to a seventh embodiment will be described referring to FIGS. 23 and 24. Incidentally, the seventh embodiment only differs in some configurations and operations as compared with the first to sixth embodiments described above, the other portions may be the same as the first to sixth embodiments. Accordingly, in the following, the description of the portions overlapping with the embodiments already described will be omitted as appropriate.

### (Functional configuration)

First, a functional configuration of the iris capturing system 10 according to the seventh embodiment will be described referring to FIG. 23. FIG. 23 is a block diagram showing the functional configuration of the iris capturing system according to the seventh embodiment. Incidentally, in FIG. 23, it is denoted by the same reference numerals to the same elements as the components shown in FIG. 2.

As shown in FIG. 23, the iris capturing system 10 according to the seventh embodiment is configured to include a capturing unit 110, an illuminance acquiring unit 120, an executing unit 130, a resolution enhancement determining unit 150 and a resolution enhancement executing unit 160 as processing blocks for realizing the functions. That is, the iris capturing system 10 according to the seventh embodiment comprises the resolution enhancement determining unit 150 and the resolution enhancement executing unit 160 in addition to the configuration of the first embodiment (see FIG. 2). Each of the resolution enhancement determining unit 150 and the resolution enhancement executing unit 160 may be implemented in the processor 11 (see FIG. 1) described above, for example.

The resolution enhancement determining unit 150 is configured to be able to determine whether or not to execute the resolution enhancement processing on the basis of the illuminance acquired by the illuminance acquiring unit 120. The resolution enhancement determining unit 150 determines that the resolution enhancement processing is executed when the circumferential resolution of the iris image becomes low due to the high illuminance. Alternatively, the resolution enhancement determining unit 150 may determine to execute the resolution enhancement processing when the radial resolution of the iris image is lowered due to the low illuminance. The resolution enhancement determining unit 150 stores, for example, a threshold value set for the illuminance, determines that the resolution enhancement processing is executed when the acquired illuminance is equal to or higher than the threshold value, and determines that the resolution enhancement processing is not executed when the acquired illuminance is less than the threshold value.

When it is determined that the resolution enhancement processing is executed by the resolution enhancement determining unit 150, the resolution enhancement executing unit 160 executes the resolution enhancement processing using the image captured by the capturing unit 110. The resolution enhancement executing unit 160 may execute the resolution enhancement processing using a single iris image or may execute the resolution enhancement processing using a plurality of iris images. The resolution enhancement executing unit 160 may execute the resolution enhancement processing considering the degree of shrinkage of the pupil. The resolution enhancement executing unit 160 may execute, for example, a resolution enhancement processing using an existing super-resolution technique. In addition, the resolution enhancement executing unit 160 may execute processing such as enlargement or sharpening, or may use a super-resolution method on the basis of deep-learning. However, the method of the resolution enhancement processing executed by the resolution enhancement executing unit 160 is not particularly limited.

### (Flow of operation)

Next, flow of operation of the iris capturing system 10 according to the seventh embodiment will be described referring to FIG. 24. FIG. 24 is a flowchart showing the flow of the operation of the iris capturing system according to the seventh embodiment. Incidentally, in FIG. 24, the same reference numerals are given to the same processing as shown in FIG. 4.

As shown in FIG. 24, when the iris capturing system 10 according to the seventh embodiment operates, first, the illuminance acquiring unit 120 acquires the illuminance around the capturing unit 110 (step S101). The illuminance acquiring unit 120 outputs the acquired illuminance to the executing unit 130.

Subsequently, the executing unit 130 executes control to bring the capturing distance closer on the basis of the illuminance acquired by the illuminance acquiring unit 120 (step S102). Then, the capturing unit 110 captures the iris image of the living body after the executing unit 130 executes the control of bringing the capturing distance closer (step S103).

Subsequently, the resolution enhancement determining unit 150 determines whether or not to execute the resolution enhancement processing on the basis of the illuminance acquired by the illuminance acquiring unit 120 (step S701). When it is determined that the resolution enhancement processing is executed (step S701: YES), the resolution enhancement executing unit 160 executes the resolution enhancement processing using the image captured by the capturing unit 110 (step S702). On the other hand, when it is determined that the resolution enhancement processing is not executed (step S701: NO), the resolution enhancement executing unit 160 does not execute the resolution enhancement S702 (that is, the processing of the step is omitted).

### (Technical effects)

Next, technical effects obtained by the iris capturing system 10 according to the seventh embodiment will be described.

As described referring to FIGS. 23 and 24, in the iris capturing system 10 according to the seventh embodiment, processing for increasing the resolution of the iris image captured by the capturing unit 110 is performed. In this way, since the resolution of the iris image is enhanced by the high resolution processing, it is possible to acquire an appropriate iris image by suppressing a decrease in resolution due to brightness.

In the above-described embodiment, in addition to the control for changing the capturing distance according to the brightness, a configuration for executing processing of resolution enhancement has been described, but without performing the control for changing the capturing distance, it may be performed processing of resolution enhancement. In other words, only resolution enhancement processing may be executed according to the brightness when capturing iris images. Even in such a case, the above-described technical effect is exhibited accordingly.

### <Eighth embodiment>

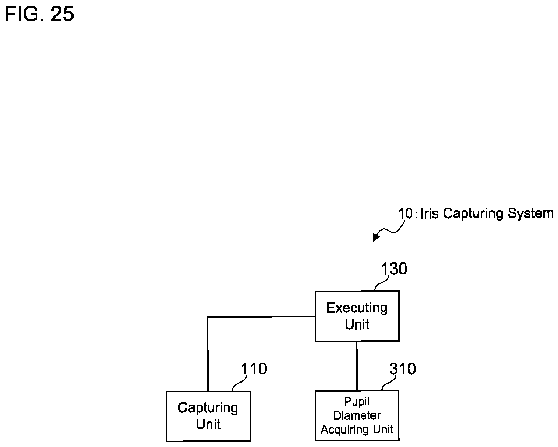

An iris capturing system 10 according to an eighth embodiment will be described referring to FIGS. 25 to 28. Incidentally, the estimation system 10 according to the eighth embodiment only differs in some configurations and operations as compared with the first to seventh forms described above, the other portions may be the same as the first to seventh embodiments. Accordingly, in the following, the description of the portions overlapping with the embodiments already described will be omitted as appropriate.

### (Functional configuration)

First, a functional configuration of the iris capturing system 10 according to the eighth embodiment will be described referring to FIG. 25. FIG. 25 is a block diagram showing the functional configuration of the iris capturing system according to the eighth embodiment. Incidentally, in FIG. 25, it is denoted by the same reference numerals to the same elements as the components shown in FIG. 2.

As shown in FIG. 25, the iris capturing system 10 according to the eighth embodiment is configured to include a capturing unit 110, a pupil diameter acquiring unit 310 and an executing unit 130 as processing blocks for realizing the functions. That is, the iris capturing system 10 according to the eighth embodiment includes a pupil diameter acquiring unit 310 instead of the illuminance acquiring unit 120 of the configuration of the first embodiment (see FIG. 2). The pupil diameter acquisition unit 310 may be implemented in, for example, the above-described processor 11 (see FIG. 1).

The pupil diameter acquiring unit 310 is configured to acquire information on the pupil diameter of the living body. Pupil diameter acquiring unit 310, for example, may acquire information relating to the pupil diameter from an image obtained by capturing the pupil of the living body. In this case, the pupil diameter acquiring unit 310 may acquire the pupil diameter from the image captured by the capturing unit 110, may acquire the pupil diameter from the image captured by another capturing unit 110. Incidentally, the image for acquiring the pupil diameter may not be an image in focus. Therefore, the pupil diameter may be obtained by using an image captured at a different position and time from the focal position of the capturing unit 110, . Incidentally, as to the specific method of obtaining the pupil diameter from the image, since the existing techniques can be appropriately adopted, a detailed description thereof will be omitted.

### (Flow of operation)

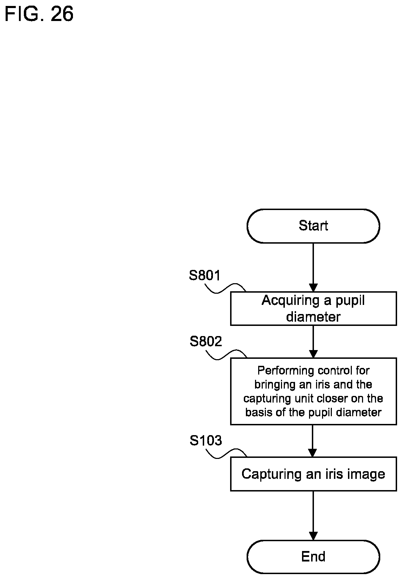

Next, flow of operation of the iris capturing system 10 according to the eighth embodiment will be described referring to FIG. 26. FIG. 26 is a flowchart showing the flow of the operation of the iris capturing system according to the eighth embodiment. Incidentally, in FIG. 26, the same reference numerals are given to the same processing as shown in FIG. 4.

As shown in FIG. 26, when the iris capturing system 10 according to the eighth embodiment operates, the pupil diameter acquiring unit 310 first acquires information on the pupil diameter of the living body (step S801). The pupil diameter acquiring unit 310 outputs the acquired pupil diameter to the executing unit 130.

Subsequently, the executing unit 130, on the basis of the pupil diameter obtained by the pupil diameter acquiring unit 310, executes a control to close the capturing distance (step S802). Executing unit 130, for example, when the pupil diameter is too small, may control so that the capturing distance is closer as the pupil diameter is smaller. Alternatively, when, for example, the pupil diameter is too large in comparison with the iris diameter, the executing unit 130 may perform control such that the larger the pupil diameter is, the closer the capturing distance is. Further, the executing unit 130, only when the pupil diameter exceeds a predetermined threshold value, may perform control to close the capturing distance.

Subsequently, the capturing unit 110 captures the iris image of the living body after the executing unit 130 executes the control of bringing the capturing distance closer (step S103). Therefore, the iris image is captured in a condition where the capturing distance is close in accordance with the pupil diameter. When it is determined that the executing unit 130 does not need to perform the control for bringing the capturing distance closer (when the pupil diameter is within a range in which bringing the capturing distance closer is unnecessary), the capturing unit 110 may capture the iris image in a condition in which the control by the executing unit 130 is not executed.

### (Modification)

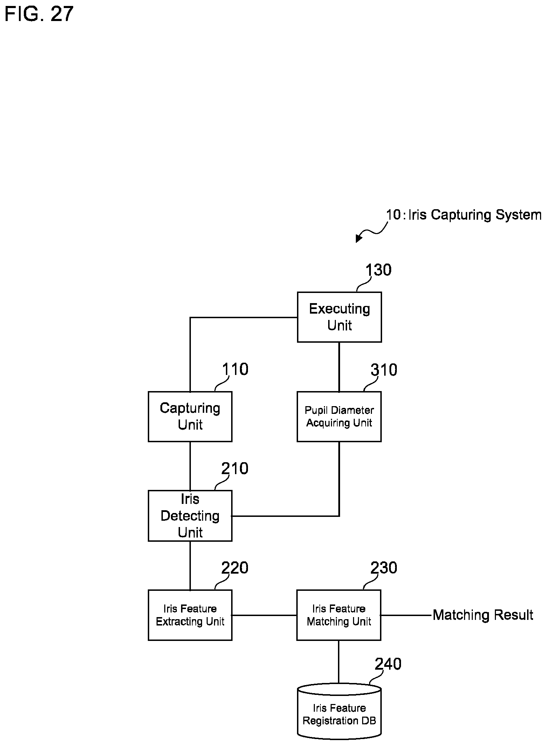

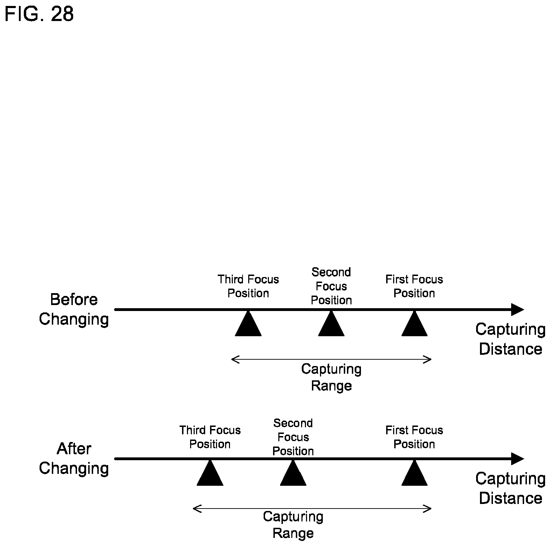

Next, an iris capturing system 10 according to a modification of the eighth embodiment will be described referring to FIGS. 27 and 28. FIG. 27 is a block diagram showing a functional configuration of an iris capturing system according to a modification of the eighth embodiment. In FIG. 27, the same reference numerals denote the same elements as the components shown in FIGS. 5 and 25. FIG. 28 is a conceptual diagram showing an example of changing the focus position by the iris capturing system according to a modification of the eighth embodiment.

Incidentally, the modification described below only differs in part of the configuration and operation as compared with the eighth embodiment, and the other portions may be the same as the eighth embodiment (see FIGS. 25 and 26). Therefore, the portions that differ from the eighth embodiment described above will be described in detail below, and the other overlapping portions will not be described as appropriate.

As shown in FIG. 27, the iris capturing system 10 according to a modification of the eighth embodiment includes a capturing unit 110, a pupil diameter acquiring unit 310, an executing unit 130, an iris detecting unit 210, an iris feature extracting unit 220, an iris feature matching unit 230 and an iris feature registering database 240 as processing blocks for realizing the functions. That is, the iris capturing system 10 according to the modified example is further configured to include the iris detection unit 210, the iris feature extracting unit 220, the iris feature matching unit 230 and the iris feature registration database 240 in addition to the configuration of the eighth embodiment (see FIG. 25).

In a modification of the eighth embodiment, in particular, the pupil diameter acquiring unit 310 acquires information about the pupil diameter on the basis of area in which the iris detected by the iris detecting unit 210 exists. In this case, the pupil diameter acquiring unit 310 may acquire the pupil diameter only at the timing at which the iris image is first acquired, or may acquire the pupil diameter each time the iris image is acquired.

Example shown in FIG. 28, as described in the sixth embodiment, the focus position changing unit 133 (see FIG. 20) is an example of changing the focus position of the capturing unit 110. In the iris capturing system 10 according to a modification of the eighth embodiment, the focus position changing unit 133 changes the focus position on the basis of the pupil diameter acquired by the pupil diameter acquiring unit 310.

In this case, the pupil diameter acquiring unit 310 first acquires the pupil diameter from the iris image captured at the first focus position. Therefore, the first capturing timing, the change of the focus position is not performed. On the other hand, for the second and third capturing, since the focus position has been changed on the basis of the pupil diameter obtained by the first capturing, the capturing distance becomes closer as compared with that before the change. Thus, when changing the focus position by acquiring the pupil diameter from the actually captured image, the first focus position is not changed, and the second and subsequent focus positions may be changed.

Further, in addition to the change of the focus position described above, by changing the intensity of the illumination, it may intentionally change the size of the pupil. For example, brightening the illumination after capturing at the first capturing timing shown in FIG. 28, the second and third capturing in a condition of contracting the pupil may be performed.

### (Technical effects)

Next, technical effects obtained by the iris capturing system 10 according to the eighth embodiment will be described.

As described referring to FIGS. 25 to 28, in the iris capturing system 10 according to the eighth embodiment, control is performed to bring the capturing distance (that is, the distance between the iris of the living body and the capturing unit 110) close to each other on the basis of the pupil diameter of the living body. As the capturing distance approaches, the iris will be imaged closer. Therefore, the resolution of the iris image is higher than that before the capturing distance is close. Therefore, according to the iris capturing system 10 according to the eighth embodiment, it is possible to capture an iris image with a high resolution in consideration of the size of the pupil.

### <Concrete Application Examples>

Next, a specific application example of an iris capturing system 10 according to the above-described embodiments will be described.

### (Entry and exit management of facilities)

The iris capturing system 10 according to the embodiments described above may be applied to a system for performing entrance/exit management of a facility. Specifically, it may be applied to a system that permits entry and exit of the target person when iris recognition is successful, and does not permit (prohibit) entry and exit of the target person when iris recognition fails. In this instance, entry and exit control of the target person may be implemented by a walk-through authentication gate and may be controlled so that the gate opens and closes in response to the outcome of the iris authentication. Then, depending on the brightness and pupil diameter when capturing, the control of changing the focus position already described (e.g., see FIGS. 21 and 22) may be implemented.

### (Payment terminal)

The iris capturing system 10 according to the embodiments described above may be applied to a system that performs a payment processing. Specifically, it may be applied to a system that operates in such a way that, if the iris authentication is successful, the payment processing is performed with a method associated with the target person, and if the iris authentication fails, the payment processing is not permitted (or other payment methods that are not associated with the target person (e.g., payments with cash) are made to be selected). In this case, depending on the brightness and pupil diameter when capturing, a control (e.g., see FIGS. 17 and 18) for guiding the standing position of the target person already described may be performed.

### (Smartphone)

The iris capturing system 10 according to the embodiments described above may be applied to various types of recognition processing in smartphones (or other portable terminals) owned by the target person. For example, an authentication processing may be performed to unlock the smartphone screen using iris images. In this case, for example, after the iris image is captured by the built-in camera of the smartphone, the lock screen may be released if the iris authentication is successful, and the lock screen may be maintained if the iris authentication fails. Since it is assumed that the smartphone is held by the target person, in order to adjust the distance between the capturing unit 110 and the target person, guidance information may be displayed so that the smartphone held in the hand can be brought close to or away from the face.

A program for operating the configuration of the embodiments to realize the functions of the embodiments described above is recorded on a recording medium, a program recorded on the recording medium is read as a code, and a processing method that is executed in a computer is also included in the scope of the embodiments. That is, a computer-readable recording medium is also included in range of the respective embodiments. In addition, not only the recording medium on which the above-described program is recorded, but also the program itself is included in each embodiment.

For example, a floppy (registered trademark) disk, a hard disk, an optical disk, a magnetic-optical disk, a CD-ROM, a magnetic tape, a non-volatile memory card, or a ROM can be used as a recording medium. In addition, not only the program recorded on the recording medium itself is executed by processing, but also the program that operates on the operating system and executes processing in collaboration with other software and expansion board functions is included in the scope of the respective embodiments.

This disclosure can be changed as appropriate in range not contrary to range of the claim and the inventive summary or philosophy which can be read from the entire specification, and iris capturing systems, iris capturing methods, and computer programs with such modifications are also included in the technical philosophy of this disclosure.

### < Supplementary notes>

With respect to the embodiments described above, it may be further described as supplementary note below, but is not limited to the following.

### (Supplementary note 1)

An iris capturing system described in a supplementary note 1 is an iris capturing system comprising: a capturing means for capturing an image including an iris of a living body; an illuminance acquiring means for acquiring brightness when capturing the image; and an execution means for executing, on the basis of the brightness when capturing the image, control for bringing a distance between the iris of the living body and the capturing means closer when capturing the image.

### (Supplementary note 2)

An iris capturing system described in a supplementary note 2 is the iris capturing system according to supplementary note 1, wherein the execution means closes the distance between the iris of the living body and the capturing means when capturing the image as an environment when capturing the image becomes brighter.

### (Supplementary note 3)

An iris capturing system described in a supplementary note 3 is the iris capturing system according to supplementary note 1 or 2, wherein the execution means estimates a pupil diameter of the living body from a brightness when capturing the image, and closes, on the basis of the estimated pupil diameter, the distance between the iris of the living body and the capturing means when capturing the image.

### (Supplementary note 4)

An iris capturing system described in a supplementary note 4 is the iris capturing system according to supplementary note 3, wherein the iris capturing system further comprises an accumulation means for accumulating information indicating a relationship between brightness when capturing the image and the pupil diameter of the living body, and the executing means estimates the pupil diameter of the living body using the information accumulated in the accumulation means.

### (Supplementary note 5)

An iris capturing system described in a supplementary note 5 is the iris capturing system according to any one of supplementary notes 1 to 4, wherein the execution means performs a notification urging movement to the living body in order to make the distance between the iris of the living body and the capturing means close when capturing the image.

### (Supplementary note 6)

An iris capturing system described in a supplementary note 6 is the iris capturing system according to any one of supplementary notes 1 to 5, wherein the execution means changes a focal position of the capturing means in order to make the distance between the iris of the living body and the capturing means close when capturing the image.

### (Supplementary note 7)

An iris capturing system described in a supplementary note 7 is the iris capturing system according to any one of supplementary notes 1 to 6, wherein the iris capturing system further comprises a resolution enhancement means for generating a high-resolution image from an image captured by the capturing means on the basis of the brightness when capturing the image.

### (Supplementary note 8)

An iris capturing system described in a supplementary note 8 is an iris capturing system comprising: a capturing means for capturing an image including an iris of a living body; a pupil diameter acquiring means for acquiring information about a pupil diameter of the living body; and an execution means for executing, on the basis of the information about the pupil diameter of the living body, control for bringing a distance between the iris of the living body and the capturing means closer when capturing the image.

### (Supplementary note 9)

An iris capturing method described in a supplementary note 9 is an iris capturing method using a capturing means for capturing an image including an iris of a living body, the iris capturing method comprising: acquiring brightness when capturing the image; and performing, on the basis of the brightness when capturing the image, control for bringing a distance between the iris of the living body and the capturing means closer when capturing the image.

### (Supplementary note 10)

A computer program described in a supplementary note 10 is a computer program for causing a computer to execute an iris capturing method using an iris capturing means for capturing an image including an iris of a living body, the iris capturing method comprising: acquiring brightness when capturing the image and performing, on the basis of the brightness when capturing the image, control for bringing a distance between the iris of the living body and the capturing means closer when capturing the image.

### (Supplementary note 11)

A recording medium described in a supplementary note 11 is a recording medium in which the computer program according to the supplementary note 10 is recorded.

### Description of Reference Numerals and Letters

10 Iris capturing system
11 Processor
20 Camera
21 Sensor
110 Capturing unit
120 Illuminance acquiring unit
130 Executing unit
131 Pupil diameter estimating unit
132 Standing position guiding unit
133 Focus position changing unit
140 Pupil diameter database
150 Resolution enhancement determining unit
160 Resolution enhancement executing unit
210 Iris detector
220 Iris feature extracting unit
230 Iris feature collation unit
240 Iris feature registration data base
250 Pupil diameter calculating unit
310 Pupil diameter acquiring unit

## Claims

1. An iris capturing system comprising:
a capturing means for capturing an image including an iris of a living body;
an illuminance acquiring means for acquiring brightness when capturing the image; and
an execution means for executing, on the basis of the brightness when capturing the image, control for bringing a distance between the iris of the living body and the capturing means closer when capturing the image.

2. The iris capturing system according to claim 1, wherein the execution means closes the distance between the iris of the living body and the capturing means when capturing the image as an environment when capturing the image becomes brighter.

3. The iris capturing system according to claim 1 or 2, wherein the execution means
estimates a pupil diameter of the living body from a brightness when capturing the image, and
closes, on the basis of the estimated pupil diameter, the distance between the iris of the living body and the capturing means when capturing the image.

4. The iris capturing system according to claim 3, wherein
the iris capturing system further comprises an accumulation means for accumulating information indicating a relationship between brightness when capturing the image and the pupil diameter of the living body, and
the executing means estimates the pupil diameter of the living body using the information accumulated in the accumulation means.

5. The iris capturing system according to any one of claims 1 to 4, wherein the execution means performs a notification urging movement to the living body in order to make the distance between the iris of the living body and the capturing means close when capturing the image.

6. The iris capturing system according to any one of claims 1 to 5, wherein the execution means changes a focal position of the capturing means in order to make the distance between the iris of the living body and the capturing means close when capturing the image.

7. The iris capturing system according to any one of claims 1 to 6, wherein the iris capturing system further comprises a resolution enhancement means for generating a high-resolution image from an image captured by the capturing means on the basis of the brightness when capturing the image.

8. An iris capturing system comprising:
a capturing means for capturing an image including an iris of a living body;
a pupil diameter acquiring means for acquiring information about a pupil diameter of the living body; and
an execution means for executing, on the basis of the information about the pupil diameter of the living body, control for bringing a distance between the iris of the living body and the capturing means closer when capturing the image.

9. An iris capturing method using a capturing means for capturing an image including an iris of a living body, the iris capturing method comprising:
acquiring brightness when capturing the image; and
performing, on the basis of the brightness when capturing the image, control for bringing a distance between the iris of the living body and the capturing means closer when capturing the image.

10. A computer program for causing a computer to execute an iris capturing method using an iris capturing means for capturing an image including an iris of a living body, the iris capturing method comprising:
acquiring brightness when capturing the image and
performing, on the basis of the brightness when capturing the image, control for bringing a distance between the iris of the living body and the capturing means closer when capturing the image.
